# EUROPEAN PATENT APPLICATION

(11) **EP 0 780 108 A1**
(43) Date of publication of application: **25.06.1997**
(21) Application number: 95120320.7
(22) Date of filing: 21.12.1995
(51) Int. Cl.: A61F 13/15

(54) **Absorbent article with apertured backsheet and fibrous super absorbent material**

(71) Applicant: THE PROCTER & GAMBLE COMPANY, Cincinnati, Ohio 45202 (US)
(72) Inventor: Coles, Peter, I-66023 Francavilla al Mare (IT); Schmidt, Mattias, D-65510 Idstein (DE)
(74) Representative: Hirsch, Uwe Thomas

(57) **Abstract**

The present invention relates to breathable absorbent articles like baby diapers, adult incontinence articles and in particular to sanitary napkins or pantyliner. According to the present invention the articles are provided with an apertured backsheet for breathability. Typically such aperture backsheets are rendered substantially liquid impermeable in order to prevent soiling through the backsheet of the article. The absorbent core of absorbent article comprises in particular super-absorbent materials which transform the absolve liquid into a solid or semi-solid form such as a gel. Such super-absorbent materials are particularly desirable for benefits to the breathable absorbent articles in which they are used. However, they are typically provided in granular form. In combination with an apertured backsheet this form is prone to a particle loss through the apertures in the backsheet. Hence the present invention relates to a breathable absorbent articles comprising non-granular super-absorbent material, preferably fibrous super-absorbent material.

## Description

### Field of the invention

The present invention relates to breathable absorbent articles like baby diapers, adult incontinence articles and in particular to sanitary napkins or pantyliner. According to the present invention the articles are provided with an apertured backsheet for breathability. The absorbent core of absorbent article comprises in particular super-absorbent materials which transform the absolve liquid into a solid or semi-solid form such as a gel. Such super-absorbent materials are particularly desirable for benefits to the breathable absorbent articles in which they are used. However, they are typically provided in granular form. In combination with an apertured backsheet this form is prone to a particle loss through the apertures in the backsheet. Hence the present invention relates to a breathable absorbent articles comprising non-granular super-absorbent material, preferably fibrous super-absorbent material.

### Background of the invention

The use of aperture backsheets for the breathability of disposable absorbent articles is well-known in the art. For example European patent applications number 94203230.1 and 94203228.5 disclose apertured film backsheets used in conjunction with fibrous non-woven materials for improved soil-through performance.

Both disclosures also mention the benefit of super-absorbent material however without disclosing or considering fibrous super-absorbent material.

On the other hand fibrous super-absorbent material has been mentioned for example in EP-A-0 336 578 which relates specifically to thin flexible sanitary napkins comprising optionally fibrous super-absorbent materials. This disclosure however lacks consideration of breathability or more specific it does not disclose any aperture in the backsheets.

WO-A-92/07534 relates and discloses fibrous super-absorbent structures. However it fails to consider breathability of disposable absorbent articles as such and in particular does not mention apertured backsheets in conjunction with the use of the absorbent structures in disposable absorbent article.

European patent application no. 94107422.1, entitled "Shields against absorbent particle loss in absorbent products" does address the problem of particle migration trough apertures in a polymer film. However this disclosure relates specifically to an aperture topsheet but not a breathable aperture backsheet. Also this disclosure solves the problem of particle migration through an apertured polymer film by including a barrier between the particle and the aperture, i.e. the use of a shield.

Based on the above the present invention addresses the problem of preventing a possible particle loss of super-absorbent material through apertures of a breathable backsheet in the context of disposable absorbent articles. It is apparent that a solution to this problem will also provide the benefit of maintaining the total absorbent capacity of the absorbent structure due to no super-absorbent particle loss, the elimination of a need for a barrier towards the apertures in the breathable backsheet and simultaneously eliminating the need for a particle loss shield between apertures in the topsheet and the super-absorbent material.

### Brief description of the invention

The present invention relates to an absorbent article comprising a topsheet having liquid passage ways, a backsheet which is rendered breathable by comprising apertures and an absorbent core interposed between the topsheet and the backsheet. The absorbent core comprises a super-absorbent material which is in a non-granular form in order to prevent super-absorbent material loss through the breathability apertures.

In particular fibrous super-absorbent materials which for example are blended with other hydrophilic fibers are desirable for the absorbent core according to the present invention. The hydrophilic fibers, with which the super-absorbent fibers can be blended, included polypropylene fibers, polyethylene fibers, polyester fibers, bicomponent fibers, cellulose fibers, modified cellulose fibers or combinations thereof. Typically the polymer fibers such as polypropylene or polyethylene need to be rendered hydrophilic by a respective treatment e.g. with surfactants.

It is preferred that the whole fibrous structure is laid down together and thermally bonded for structural integrity by an air-through-process. This also is capable of providing a liquid stable void volume in the absorbent structure whereas unbonded fibers structures typically would collapse upon being wetted.

It is particularly preferred to use the present invention if the apertures in the backsheet are provided in an extensible part of the backsheet. Extensible backsheets with the breathability apertures have the disadvantage that upon extending the whole or a part of the backsheet, the size of the apertures in the region of extension also increase and hence the probability of super-absorbent particle loss (if such particles were present) would raise. Particularly preferred are extensible backsheet materials which are also elastic, i.e. which return fully or partially to their original size after the extending forces are removed.

Particularly preferred embodiments according to the present invention are absorbent articles in which the non-granular super-absorbent material, typically the fibrous super-absorbent material, is in direct contact with the backsheet such that no additional layers between the backsheet and the non-granular super-absorbent material exists. Obviously the same is true for the contact between the non-granular super-absorbent material and the topsheet. The most preferred absorbent articles according to the present invention are sanitary napkins, incontinence inserts or pantyliners in which breathability is usually highly desirable but which at the same time need to provide sufficient absorbency and hence often are provided with super-absorbent materials.

### Detailed description of the invention

### Definitions

As used herein, the term "aqueous body fluids" includes urine, menses and vaginal discharges.

As used herein, the term "absorbent product" refers to articles which are capable of absorbing aqueous body fluids like disposable baby, children or adult diapers, incontinence products like inserts or bed pads and sanitary napkins.

As used herein, the term "sanitary napkin" refers to an absorbent article that is worn by females adjacent to the pudendal region, generally external to the urogenital region, and which is intended to absorb and contain aqueous body fluids and other vaginal discharges from the wearer's body. It should be understood, however, that the present invention is also applicable to other feminine hygiene or catamenial pads such as pantyliners, or other absorbent articles such as incontinence pads, and the like.

As used herein the term "granular" refers to particles of uniform size, particles having a size distribution including micro particles (e.g. smaller than 0.2 mm diameter) and agglomerated particles. The term "non-granular" excludes those materials which are granular.

### Detailed description of the invention

According to the present invention the absorbent product comprises three main elements: a topsheet, a core and a backsheet. Typically the topsheet is worn next to the skin of the user while the backsheet is worn next to the undergarment of the user with the core interposed between both. Each of the three main elements of the absorbent product can be selected from a wide variety of alternatives and can also comprise several elements and/or layers contributing to the individual function of each of the main elements.

In the following non-limiting embodiments of the main elements of the absorbent product are described.

### Absorbent core

This absorbent core can be a single entity or preferably comprise several layers. It can includes the following components: (a) optionally a primary fluid distribution layer; (b) optionally, but preferably, a secondary fluid distribution layer; (c) a fluid storage layer; (d) optionally a fibrous layer underlying the storage layer; and (e) other optional components.

### a. Primary Fluid Distribution Layer

One optional component of the absorbent core according to the present invention is the primary fluid distribution layer. This primary distribution layer typically underlies the topsheet and is in fluid communication therewith. The topsheet transfers the acquired menstrual fluid to this primary distribution layer for ultimate distribution to the storage layer. This transfer of fluid through the primary distribution layer occurs not only in the thickness, but also along the length and width directions of the absorbent product.

### b. Optional Secondary Fluid Distribution Layer

Also optional but preferred component of the absorbent cores according to the present invention is a secondary fluid distribution layer. This secondary distribution layer typically underlies the primary distribution layer and is in fluid communication therewith. The purpose of this secondary distribution layer is to readily acquire menstrual fluid from the primary distribution layer and transfer it rapidly to the underlying storage layer. This helps the fluid capacity of the underlying storage layer to be fully utilized.

### c. Fluid Storage Layer

Positioned in fluid communication with , and typically underlying the primary or secondary distribution layers, is a fluid storage layer comprising super-absorbent gelling materials usually referred to as "hydrogels," "superabsorbent" "hydrocolloid" materials. Absorbent gelling materials are those materials that, upon contact with aqueous fluids, especially aqueous body fluids, imbibes such fluids and thus form hydrogels. These absorbent gelling materials are typically capable of absorbing large quantities of aqueous body fluids, and are further capable of retaining such absorbed fluids under moderate pressures. In the prior art these absorbent gelling materials are also typically in a granular form of discrete, nonfibrous particles. However, according to the present invention these super-absorbent gelling materials are provided in non-granular form, preferably in a fibrous form.

This fluid storage layer can comprise solely absorbent gelling materials, or these absorbent gelling materials can be dispersed homogeneously or non-homogeneously in a suitable carrier. Suitable carriers include cellulose fibers, in the form of fluff, such as is conventionally utilized in absorbent cores. Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferred synthetic fibers have a denier of from about 3 denier per filament to about 25 denier per filament, more preferably from about 5 denier per filament to about 16 denier per filament. Also preferably, the fiber surfaces are hydrophilic or are treated to be hydrophilic. The storage layer can also include filler materials, such as Perlite, diatomaceous earth, Vermiculite, etc., that lower rewet problems.

If dispersed non-homogeneously in a carrier, the storage layer can be locally homogeneous, i.e. have a distribution gradient in one or several directions within the dimensions of the storage layer. Non-homogeneous distribution can also refer to laminates of carriers enclosing absorbent gelling materials.

Generally, the storage layer comprises from about 15 to 100% absorbent gelling materials and from 0 to about 85% carrier. Preferably, the storage layer comprises from about 30 to 100 %, most preferably from about 60 to 100% absorbent gelling materials and from 0 to about 70 %, most preferably from 0 to about 40 %, carrier.

Suitable absorbent gelling materials for use herein will most often comprise a substantially water-insoluble, slightly crosslinked, partially neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 (Brandt et al), issued March 31, 1987, and reissued as RE 32,649 on April 19, 1988. Preferred monomers include acrylic acid, methacrylic acid, and 2-acrylamido-2-methyl propane sulfonic acid. Acrylic acid itself is especially preferred for preparation of the super-absorbent material. The polymeric component formed from the unsaturated, acid-containing monomers can be grafted onto other types of polymer moieties such as starch or cellulose. Polyacrylate grafted starch materials of this type are especially preferred. Preferred polymeric absorbent gelling materials that can be prepared from conventional types of monomers include hydrolyzed acrylonitrile grafted starch, polyacrylate grafted starch, polyacrylates, maleic anhydride-based copolymers and combinations thereof. Especially preferred are the polyacrylates and polyacrylate grafted starch.

Whatever the nature of the basic polymer components of the hydrogel-forming polymeric absorbent gelling materials, such materials will in general be slightly crosslinked. Crosslinking serves to render the hydrogel-forming polymer gelling materials substantially water-insoluble, and cross-linking thus in part determines the gel volume and extractable polymer characteristics of the hydrogels formed from these polymeric gelling materials. Suitable crosslinking agents are well known in the art and include, for example, those described in greater detail in U.S. Patent 4,076,663 (Masuda et al), issued February 28, 1978. Preferred crosslinking agents are the di- or polyesters of unsaturated mono- or polycarboxylic acids with polyols, the bisacrylamides and the di- or triallyl amines. Other preferred crosslinking agents are N,N'-methylenebisacrylamide, trimethylol propane triacrylate and triallyl amine. The crosslinking agent can generally constitute from about 0.001 mole percent to 5 mole percent of the resulting hydrogel-forming polymer material. More preferably, the crosslinking agent will constitute from about 0.01 mole percent to 3 mole percent of the hydrogel-forming polymeric gelling material.

The slightly crosslinked, hydrogel-forming polymeric gelling materials are generally employed in their partially neutralized form. For purposes of the present invention, such materials are considered partially neutralized when at least 25 mole per-cent, and preferably at least 50 mole percent of monomers used to form the polymer are acid group-containing monomers that have been neutralized with a salt-forming cation. Suitable salt-forming cations include alkali metal, ammonium, substituted ammonium and amines. This percentage of the total monomers utilized which are neutralized acid group-containing monomers is referred to herein as the "degree of neutralization."

While these absorbent gelling materials have typically been disclosed in the prior art in granular form, it is required by the present invention that the absorbent gelling material is in a non-granular form for example as macrostructures such as fibers, sheets or strips. These macrostructures can be prepared by forming the particulate absorbent gelling material into an aggregate, treating the aggregated material with a suitable crosslinking agent, compacting the treated aggregate to densify it and form a coherent mass, and then curing the compacted aggregate to cause the crosslinking agent to react with the particulate absorbent gelling material to form a composite, porous absorbent macrostructure. Such porous, absorbent macrostructures are disclosed, for example, in U.S. Patent 5,102,597 (Roe et al), issued April 7, 1992.

More generally any super-absorbent fiber known in the art may be used as a super-absorbent fiber in the absorbent core. Superabsorbent fibers can be made by forming a water soluble super-absorbent polymer into water soluble filaments, contacting the filaments with a primary air stream having a velocity effective to attenuate and to partially dry the filaments, and contacting the attenuated filaments with a secondary air stream having a velocity effective to fragment the filaments into fibers. Particularly suitable super-absorbent polymers are polymers comprising a blend of
- a copolymer of at lest one alpha, beta-unsaturated carboxylic monomer and at least one monomer copolymerizable therewith, and
- a cross-linking agent having cross-linking functionality comprising hydroxyl or heterocyclic carbonated groups. Preferred are maleic anhydrede/isobutylene copolymers cross-linked with propylene carbonate or a mixture of pentaerythriol and butanediol.

An example of a super-absorbent fiber embodiment of the present invention is Fibersorb, a commercially available superabsorbent fiber from Arco Chemical Company of Newton Square, Pennsylvania. These fibers are disclosed more fully in U.S. Patent No. 4,855,179, issued August 8, 1989, to Bourland et al.

### d. Optional Fibrous ("Dusting") Layer

An optional component for inclusion in the absorbent cores according to the present invention is a fibrous layer adjacent to, and typically underlying the storage layer. This underlying fibrous layer is typically referred to as a "dusting" layer since it provides a substrate on which to deposit the storage layer during manufacture of the absorbent core. In a preferred embodiment this fibrous "dusting" layer need not be included because the absorbent gelling material is in the form of macrostructures such as fibers, sheets or strips.

### e. Other Optional Components

The absorbent cores according to the present invention can include other optional components normally present in absorbent webs. For example, a reinforcing scrim can be positioned within the respective layers, or between the respective layers, of the absorbent cores. Such reinforcing scrims should be of such configuration as to not form interfacial barriers to fluid transfer, especially if positioned between the respective layers of the absorbent core. Given the structural integrity that usually occurs as a result of thermal bonding, reinforcing scrims are usually not required for the absorbent structures according to the present invention.

Another component which can be included in the absorbent core according to the invention and preferably is provided close to or as part of the primary or secondary fluid distribution layer are odor control agents. Typically active carbon coated with or in addition to other odor control agents, in particular suitable zeolite or clay materials, are optionally incorporated in the absorbent core. These components can be incorporated in any desired form but are preferably included also in non-granular form or enclosed in a particle migration hindering structure.

### Physical characteristics of absorbent cores

Absorbent cores are usually non extensible and non-elastic, however, they can be rendered extensible and depending on the selected materials can also be made to have elastic characteristics. The term "extensible" as used hereinafter refers to a structure which under external forces such as those accuring during use of a disposable absorbent article, in particular a sanitary napkin, extends in the direction of the forces or in the direction of a component of the forces in cases where only mono directional extensibility is provided.

The term "elastic" as used hereinafter refers to extensible structures which return at least partially to their initial state after the forces causing the extension seize to be excerted. Absorbent structures can be corrugated or pleated in one or several directions to provide a certain extensibility while selection of elastic fibers for the structure can provide elasticity.

The absorbent cores should preferably be thin. In the case of sanitary napkins a thickness of less than 12 mm, preferably less than 5 mm is desirable, while panty liners should have a thickness of preferably less than 3 mm.

### Topsheet

The topsheet is compliant, soft feeling, and non-irritating to the wearer's skin. The topsheet also can have elastic characteristics allowing it to stretch in one or two directions. Further, the topsheet is fluid pervious permitting fluids (e.g., menses and/or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present are selected from high loft nonwoven topsheets and aperture formed film topsheets. Apertured formed films are especially preferred for the topsheet because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135 (Thompson), issued December 30, 1975; U.S. Patent 4,324,246 (Mullane, et al.), issued April 13, 1982; U.S. Patent 4,342,314 (Radel. et al.), issued August 3, 1982; U.S. Patent 4,463,045 (Ahr et al.), issued July 31, 1984; and U.S. 5,006,394 (Baird), issued April 9, 1991. Particularly preferred microapetured formed film topsheets are disclosed in U.S. patent 4,609,518 (Curro et al), issue September 2, 1986 and U.S. patent 4,629,643 (Curro et al), issued December 16, 1986. The preferred topsheet for the present invention is the formed film described in one or more of the above patents and marketed on sanitary napkins by The Procter & Gamble Company of Cincinnati, Ohio as "DRI-WEAVE."

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer through the topsheet faster than if the body surface was not hydrophilic. In a preferred embodiment, surfactant is incorporated into the polymeric materials of the formed film topsheet such as is described in U.S. Patent Application Serial No. 07/794,745, "Absorbent Article Having A Nonwoven and Apertured Film Coversheet" filed on November 19, 1991 by Aziz, et al. Alternatively, the body surface of the topsheet can be made hydrophilic by treating it with a surfactant such as is described in the above referenced U.S. 4,950,254.

### Backsheet

The backsheet prevents the exudates absorbed and contained in the absorbent core from wetting articles that contact the sanitary napkin such as pants, pajamas and undergarments. Preferably the backsheet is impervious to liquids (e.g., menses and/or urine) and is preferably manufactured from a modified thin plastic film, although other flexible liquid impervious materials can also be used. As used herein, the term "flexible" refers to materials that are compliant and will readily conform to the general shape and contours of the human body. The backsheet preferably also can have elastic characteristics allowing it to stretch in one or two directions.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance.

The articles according to the present invention have a breathable backsheet comprising apertures.

The backsheet can also comprise more than one breathable layer so as to replace a single breathable backsheet layer by at least two layers of a different or the same material. In particular two breathable layers forming together the breathable backsheet are preferred.

In an embodiment of the present invention the breathable backsheet comprises a hydrophobic, gas-permeable fibrous fabric layer composed of polymeric fibres. Such fibrous layers have inherently breathability apertures but in addition may be provided with apertures such as the film described below. In an alternative embodiment of the present invention the breathable backsheet comprises a hydrophobic, gas-permeable apertured polymeric film. This film preferably has a first liquid transport direction and a second liquid transport direction opposite the first liquid transport direction and is oriented such that the first direction is from the backsheet towards the absorbent core. This directional apertured film allows a liquid transport in the first liquid transport direction which is larger than the liquid transport in the second liquid transport direction under an identical pressure drop across said apertured film. Most preferably the film comprises funnel shaped apertures wherein the direction from the larger funnel opening towards the smaller funnel opening is parallel to the first liquid transport direction.

The fibrous fabric layer useful in the present invention preferably has a basis weight of 10 to 100 g/m² preferably 15 to 30 g/m². The fibres can be made of any hydrophobic polymeric material usual in the art of making fibrous fabric layers. Depending on the circumstances of the ultimate use and manufacturing of the breathable absorbent article fibres of polyethylene, polypropylene, polyester, polyacetat or combinations thereof (intra- and inter-fibres combinations) have been found useful.

The fibres are preferably spunbonded, carded or melt blown. The fabric layer most preferably comprises a matrix of spunbonded fibres covered on one or both sides with meltblown fibres but can also be provided by any other typical technology used in the art. If two or more fibrous fabric layers of different basis weight are used it is most preferred to have the one with the highest basis weight on the outside of the absorbent article.

The apertured film also useful for embodiments of the present invention can be any of those well known in the art. This includes in particular, but is not limited to those films disclosed in U.S. 3,929,135, U.S. 4,151,240, U.S. 4,319,868, U.S. 4,324,426, U.S. 4,342,314 , U.S. 4,591,523 and U.S. 4,609,518, U.S. 4,629,643, U.S.5,158,819, U.S. 4,772,444.

The apertured film comprised in the inner layer of the breathable backsheet preferably has funnel shaped apertures similar to those described e. g. in US 3,929,135. The apertures maybe circular or non-circular but have a cross sectional dimension at one end of the funnel which is wider than the opening at the other end of the funnel. The direction from the larger funnel opening towards the smaller opening is of course parallel to the first liquid transport direction. The open area of the apertured film is typically more than 5 %, preferably in the range of 10 % to 35 % of the total film surface. The apertured films can be made of any material typical in the art but preferably is made of a polymer similar to those used for the fibrous fabric layer.

The minimum hydraulic diameter of the apertures in the film should be as small as possible while still providing sufficient gas permeability without hydraulic blockage of the apertures. A hydraulic diameter of as little as 0.1 mm, preferably 0.2 to 0.7 mm has been found possible in the context of the present invention. Hydraulic diameter for non circular apertures is the diameter that a circular aperture with the same cross section would have. Diameter is always determined in the plane of smallest cross section of an aperture.

The present invention as indicated above can be used beneficially in the context of many different absorbent articles. However sanitary napkins and especially thin panty liners are particularly susceptible to the present invention. Sanitary napkins or panty liners having a thickness of 3 mm or less and preferably 2 mm or less benefit especially well from the breathable backsheet of the present invention.

### Optional components of the absorbent products

Optionally, the absorbent product of the present invention can comprise all those components typical for the particularly intended product use. For example catamenials, panty liners and sanitary napkins often comprise components such as wings and panty fastening adhesives in order to improve their positioning and soiling protection performance. Baby diapers comprise adhesive or mechanical closure systems such as tapes and dedicated fastening surfaces or velcro (TM) systems. Elasticated waistbands, waistbelts and other waist features are also common in baby diapers or adult incontinence products. Leg elastication by one or several elastic strands is also common in the art of absorbent products. In general, all typically used components in absorbent products can also be comprised in the absorbent products according to the present invention as long as the article comprises an apertured breathable backsheet and a non-granular super-absorbent material.

## Claims

1. An absorbent article comprising
a topsheet having liquid passage ways;
a backsheet, said backsheet being rendered breathable by comprising breathability apertures; and
an absorbent core interposed between said topsheet and said backsheet, said core comprising a super-absorbent material;
said article being characterised in that
said super-absorbent material is non-granular to prevent super-absorbent material loss through said breathability apertures.

2. An absorbent article according to claim 1 characterised in that said super-absorbent material is fibrous.

3. An absorbent article according to any of the preceding claim wherein said absorbent core further comprises hydrophilic fibers blended with said super-absorbent material.

4. An absorbent article according to claim 3 characterised in that said hydrophilic fibers comprise polypropylene fibers, polyethylene fibers, polyester fibers, bicomponent fibers, cellulose fibers, modified cellulose fibers or combinations thereof.

5. An absorbent article according to claim 3 characterised in that said super-absorbent material is fibrous, said hydrophilic fibers and said super-absorbent fibers are laid down together and are thermally bonded together by an air-through-process.

6. An absorbent article according to any of the preceding claims characterised in that said breathability apertures through said backsheet are located in an extensible portion of said backsheet.

7. An absorbent article according to any of the preceding claims characterised in that said backsheet comprises an elastically extensible material.

8. An absorbent article according to any of the preceding claims characterised in that said non-granular super-absorbent material is in direct contact with said backsheet.

9. An absorbent article according to claim 8 characterised in that said non-granular super-absorbent material is in direct contact with said topsheet.

10. An absorbent article according to any of the preceding claims characterised in that said article is a sanitary napkin, incontinence insert or pantyliner.
